(19) Europäisches Patentamt
European Patent Office
Office européen des brevets

(11) EP 1 699 497 B1

# (12) EUROPEAN PATENT SPECIFICATION

(45) Date of publication and mention of the grant of the patent:
**15.04.2009 Bulletin 2009/16**

(21) Application number: **04815734.1**

(22) Date of filing: **28.12.2004**

(51) Int Cl.:
***A61L 12/08*** *(2006.01)* ***A45C 11/00*** *(2006.01)*
***G02B 1/04*** *(2006.01)* ***G02C 7/04*** *(2006.01)*

(86) International application number:
**PCT/US2004/043722**

(87) International publication number:
**WO 2005/065731 (21.07.2005 Gazette 2005/29)**

## (54) ANTIMICROBIAL CONTACT LENSES AND METHODS FOR THEIR PRODUCTION

ANTIMIKROBIELLE KONTAKTLINSEN UND ZUGEHÖRIGE HERSTELLUNGSVERFAHREN

VERRES DE CONTACT ANTIMICROBIENS ET PROCEDES DE PRODUCTION DE CES VERRES

(84) Designated Contracting States:
**DE FR GB IE IT**

(30) Priority: **30.12.2003 US 748621**

(43) Date of publication of application:
**13.09.2006 Bulletin 2006/37**

(73) Proprietor: **Johnson and Johnson Vision Care, Inc.**
**Jacksonville, FL 32256 (US)**

(72) Inventors:
- **NEELY, Frank**
**Jacksonville, Florida 32259 (US)**
- **ALLI, Azaam**
**Jacksonville, Florida 32221 (US)**

(74) Representative: **Fisher, Adrian John et al**
**CARPMAELS & RANSFORD**
**43-45 Bloomsbury Square**
**London WC1A 2RA (GB)**

(56) References cited:
**WO-A-02/49683**
**WO-A-03/011351**
**DE-A1- 10 024 363**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 699 497 B1

## Description

FIELD OF THE INVENTION

This invention relates to contact lenses having antimicrobial properties as well as methods of their production.

BACKGROUND OF THE INVENTION

Contact lenses have been used commercially to improve vision since the 1950s. The first contact lenses were made of hard materials. Although these lenses are currently used, they are not suitable for all patients due to their poor initial comfort and their relatively low permeability to oxygen. Later development in the field gave rise to soft contact lenses, based upon hydrogels, which are extremely popular today. Many users find soft lenses are more comfortable, and increased comfort levels allow soft contact lens users to wear their lenses for far longer hours than users of hard contact lenses.

Despite this advantage, the extended use of the lenses can encourage the buildup of bacteria or other microbes, particularly, *Pseudomonas aeruginosa,* on the surfaces of soft contact lenses. The build-up of bacteria or other microbes is not unique to soft contact lens wearers and may occur during the use of hard contact lenses as well.

Therefore, there is a need to produce contact lenses that inhibit the growth of bacteria or other microbes and/or the adhesion of bacterial or other microbes on the surface of contact lenses. Further there is a need to produce contact lenses which do not promote the adhesion and/or growth of bacteria or other microbes on the surface of the contact lenses. Also there is a need to produce contact lenses that inhibit adverse responses related to the growth of bacteria or other microbes.

WO02/49683 discloses contact lenses comprising silver and a polymer formed from the monomer of formula I below.

Although methods and lenses are known, other contact lenses that inhibit the growth and/or adhesion of bacteria or other microbes and are of sufficient optical clarity, as well as methods of making those lenses are still needed. It is this need, which this invention seeks to meet.

DETAILED DESCRIPTION OF THE INVENTION

This invention includes an antimicrobial lens having improved antimicrobial efficacy. Specifically, the lenses of the present invention have metal to ligand weight ratio of greater than 0.6, and preferably 0.8.

The lenses of the present invention are defined in claim 1 and comprise, consist essentially of, or consist of, silver and a silicone elastomer or hydrogel formed from a reaction mixture comprising at least one ligand monomer of Formula I

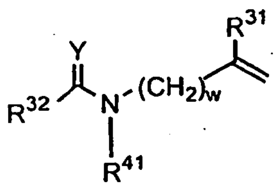

I

The ligand monomers in accordance with the invention are monomers where
w is 0-1;
$R^{31}$ is hydrogen;
$R^{32}$ is amine, $C_{1-3}$alkylamine, phenylamine, substituted phenylamine, thio$C_{1-3}$alkylcarbonyl;
$R^{41}$ is hydrogen, or
the following monomers

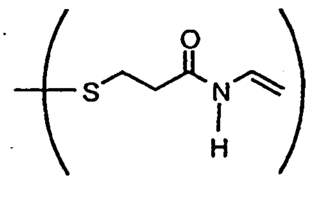

2,

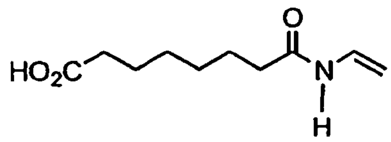

,

and

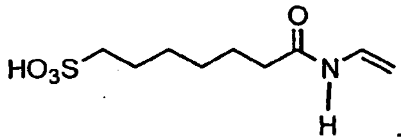

.

Mixtures of ligand monomers may also be used. In a particularly preferred embodiment the at least one ligand monomer comprises 1-allyl-2-thiourea.

As used herein, the term "lens" refers to opthalmic devices that reside in or on the eye. These devices can provide optical correction or may be cosmetic. The term lens includes but is not limited to soft contact lenses, intraocular lenses, overlay lenses, ocular inserts, and optical inserts. Soft contact lenses are made from silicone elastomers or hydrogels, which include but are not limited to silicone hydrogels and fluorohydrogels. These hydrogels may be formed from lens forming components, including hydrophobic and/or hydrophilic monomers that are covalently bound to one another in the cured lens.

As used herein the term "polymers" means copolymers, homopolymers, or mixtures thereof. The ligand monomers or their homopolymers, are added to the monomer mix of contact lenses, prior to polymerization in an amount based on the weight percent of the initial monomer mix, including a suitable diluent if said diluent is used in the preparation of the polymer. The weight percentage of the ligand monomers of the invention can vary with the lens formulation. The maximum percentage of ligand monomers is the percentage that does not compromise the physical properties of the resulting contact lens, such as, but not limited to modules, of the resulting lens. The minimum percentage of ligand monomer is an amount that allows the incorporation of a sufficient amount of silver into a lens to provide the desired antimicrobial effect. Preferably, about 0.01 to about 20.0 weight percent of at least one ligand monomer is added, to a monomer mix, more preferably, about 0.01 to about 1.5 weight percent, even more preferably, about 0.01 to about 0.4 weight percent, most preferably, about 0.05 to about 0.2 weight percent, all based upon the total lens forming components in the monomer mix.

Suitable lens forming components are known in the art and include acrylic- or vinyl-containing monomers, hydrophobic monomers and macromers internal wetting agents and compatibllizing monomers and macromers, initiators. UV absorbing compounds, visibility tints, crosslinkers combinations thereof and the like. Acrylic-containing monomers contain the acrylic group: ($CH_2$=CRCOX-) wherein R is H or $CH_3$, and X is O or N, polymerize readily and include, but are not limited to N,N-dimethyl acrylamide (DMA), 2-hydroxyethyl methacrylate (HEMA), glycerol methacrylate, 2-hydroxyethyl methacrylamide, polyethyloneglycol monomethacrylate, methacrylic acid and acrylic acid.

Vinyl-containing monomers contain the vinyl grouping (-CH=$CH_2$), and include but are not limited to monomers such as N-vinyl lactams (such as, but not limited to N-vinylpyrrolidone, or NVP), N-vinyl-N-methyl acetamide, N-vinyl-N-ethyl acetamide, N-vinyl-N-ethyl formamide, N-vinyl formamide, with NVP being preferred.

As used herein the term "compatibilizing monomers and macromers" mean reaction components which contain at least one silicone group and at least one hydroxyl group. Such components have been disclosed in US 6,367,929, WO03/022321 and WO03/022322, along with any other patents or applications which are referenced herein. A suitable example includes 3-methacryloxy-2-hydroxypropyloxypropylbis(trimethylsiloxy)methylsilane.

Suitable hydrophobic components include silicone containing components and fluorine containing components. Silicone-containing components contain at least one [-Si-O-Si] group, and at least one polymerizable functional group in a monomer, macromer or prepolymer. Preferably, the Si and attached O are present in the sillcone-containing component in an amount greater than 20 weight percent, and more preferably greater than 30 weight percent of the total molecular weight of the silicone-containing component. Examples of silicone-containing components which are useful in this invention may be found in U.S. 3.808,178; 4,120,570; 4,136,250; 4,153,641; 4,740,533; 5,034,461. 5,070,215, WO03/022322, WO03/022321. US 6,367,929, US 5,998,498, 5,760,100, 5,260,000, 4,719,943, 4,139,593, US

4,139,548. US 4,235,985 and EP080539. Examples of suitable hydrophobic monomers include, but are not limited to tris(trimethylsiloxy)silylpropyl methacrylate, monomethacryloxypropyl terminated polydimethylsiloxanes, polydimethylsiloxanes, 3-methacryloxypropylbis(trimethylsiloxy)methylsilane, methacryloxypropylpentamethyl disiloxane, N-tris(trimethylsiloxy)-silylpropylmethacrylamide, N-tris(trimethylsiloxy)-silylpropylacrylamide and combinations thereof.

Silicone hydrogels of the present invention may also include an internal wetting agent, such as, but not limited to at least one "high molecular weight hydrophilic polymer", which refers to substances having a weight average molecular weight of no less than about 100,000 Daltons, wherein said substances upon incorporation to silicone hydrogel formulations, increase the wettability of the cured silicone hydrogels. Suitable high molecular weight hydrophilic polymers are disclosed in WO03/022321.

Suitable amounts of high molecular weight hydrophilic polymer include from about 1 to about 15 weight percent, more preferably about 3 to about 15 percent, most preferably about 3 to about 12 percent, all based upon the total of all lens forming components.

Examples of high molecular weight hydrophilic polymers include but are not limited to polyamides, polylactones, polyimides, polylactams and functionalized polyamides, polylactones, polyimides, polylactams. Hydrophilic prepolymers made from DMA or n-vinyl pyrrolidone with glycidyl methacrylate may also be used. The glycidyl methacrylate ring can be opened to give a diol which may be used in conjunction with other hydrophilic prepolymer in a mixed system to increase the compatibility of the high molecular weight hydrophilic polymer, hydroxyl-functionalized silicone containing monomer and any other groups which impart compatibility. The preferred high molecular weight hydrophilic polymers are those that contain a cyclic moiety in their backbone, more preferably, a cyclic amide or cyclic imide. High molecular weight hydrophilic polymers include but are not limited to poly-N-vinyl pyrrolidone, poly-N-vinyl-2- piperidone, poly-N-vinyl-2-caprolactam, poly-N-vinyl-3-methyl-2-caprolactam, poly-N-vinyl-3-methyl-2-piperidone, poly-N-vinyl-4-methyl-2-piperidone, poly-N-vinyl-4-methyl-2-caprolactam, poly-N-vinyl-3-ethyl-2-pyrrolidone, and poly-N-vinyl-4,5-dimethyl-2-pyrrolidone, polyvinylimidazole, poly-N-N-dimethylaaylamide, polyvinyl alcohol, polyacrylic acid, polyethylene oxide, poly 2 ethyl oxazoline, heparin polysaccharides, polysaucharides, mixtures and copolymers (including block or random, branched, multichain, comb-shaped or star shaped) thereof where poly-N-vinylpyrrolidone (PVP) is preferred.

Other lens forming components such as crosslinkers, UV absorbing agents, tinting agents are known in the art and need not be described here.

The type of initiator used In the present invention is not critical. Suitable intitiators include thermal initators such as lauryl peroxide, benzoyl peroxide, isopropyl percarbonate, azobisisobutyronitrile, and the like, that generate free radicals at moderately elevated temperatures, and photoinitiator systems such as aromatic alpha-hydroxy ketones, alkoxyoxybenzoins, acetophenones, acylphosphine oxides, bisacylphosphine oxides, and a tertiary amine plus a diketone, mixtures thereof and the like. Illustrative examples of photoinitiators are 1-hydroxycyclohexyl phenyl ketone, 2-hydroxy-2-methyl-1-phenyl-propan-1-one, bis(2,6-dimethoxybenzoyl)-2,4-4-trimethylpentyl phosphine oxide (DMBAPO), bis(2,4,6-trimethylbenzoyl)-phenyl phosphineoxide (Irgacure 819), 2,4,6-trimethylbenzyldiphenyl phosphine oxide and 2.4,6-trimethylbenzoyl diphenylphosphine oxide, benzoin methyl ester and a combination of camphorquinone and ethyl 4-(N,N-dimethylamino)benzoate. Commercially available visible light initiator systems include Irgacure 819, Irgacure 1700, Irgacure 1800, Irgacure 1850 (all from Ciba Specially Chemicals) and Lucirin TPO initiator (available from BASF). Commercially available UV photoinitiators include Darocur 1173 and Darocur 2959 (Ciba Specialty Chemicals). These and other photoinitiators which may be used are disclosed in Volume III, Photoinitiators for Free Radical Cationic & Anionic Photopolymerization, 2nd Edition by J.V. Crivello& K. Dietliker, edited by G. Bradley; John Wiley and Sons; New York; 1998.

The ligand monomers or their homopolymers, are mixed with the lens forming components in a diluent, prior to polymerization in an amount based on the weight percent of the initial monomer mix, including a suitable diluent if said diluent is used in the preparation of the polymer. The weight percentage of the ligand monomers can vary with the lens formulation. The maximum percentage of ligand monomers is the percentage that does not compromise the physical properties of the resulting contact lens, such as, but not limited to, modulus of the resulting lens. The minimum percentage of ligand monomers is an amount that allows the incorporation of a sufficient amount of silver into a lens to provide the desired antimicrobial effect. Preferably, about 0.01 to about 20.0 weight percent (based upon the total weight of lens forming components and ligand monomer) of ligand monomers is added, to a contact lens formulation, more preferably, about 0.01 to about 3 weight percent, and in some embodiments as little as 100 ppm to about 2000 ppm may be added.

Ligand monomers are added to the soft contact lens formulations described in U.S. Pat. No. 5,710,302, WO 9421698, EP 406161, JP 2000016905, U.S. Pat. No. 5,998,498, WO03/022322, WO03/022321, 5,760,100. 5,260,000 and U.S. 6,087,415.. In addition, ligand monomers may be added to the formulations of commercial soft contact lenses. Examples of commercially available soft contact lenses formulations include but are not limited to, the formulations of etafilcon A, genfilcon A, lenefilcon A, polymacon, acquafilcon A, balafilcon A, senofilcon A, galyflicon A and lotrafilcon A. The preferable contact lens formulations are etafilcon A, balafilcon A, lotrafilcon A, senofilcon A, galyfilcon A and silicone hydrogels, as prepared in U.S. 5,760,100; U.S. 5,776,999; U.S. 5.849,811; U.S. 5,789,461; U.S. 5.998.498. WO03/022321, WO03/02232 and 10/236,762, and U.S. 6,087,415.

Lenses prepared from the aforementioned formulations and the ligand monomers may be coated with a number of agents that are used to coat lenses. For example, the procedures, compositions, and methods of U.S. 3,854,982; 3,916,033; 4,920,184; and 5,002,794; 5,712,327; and 6,087,415 as well as WO 0127662, WO03/011551, may be used. In addition to the cited coating patents, there are other methods of treating a lens once it is formed. The lenses of this invention may be treated by these methods and the following publications illustrate these methods: U.S. 5,453,467; U.S. 5,422,402; WO 9300391; U.S. 4,973.493; and U.S. 5,350,800.

As used herein, the term "silver" refers to silver ions that are incorporated into a lens. While not wanting to be bound as to the oxidation state of the silver ($Ag^{1+}$or $Ag^{2+}$), that is incorporated into the lens, silver may be added to the lens by washing the cured and hydrated lens in a silver solution such as silver nitrate In deionized water ("DI"). Other sources of silver include but are not limited to silver acetate, silver citrate, silver iodide, silver lactate, silver picrate, and silver sulfate. The concentration of silver in these solutions can vary from the concentration required to add a known quantity of silver to a lens to a saturated silver solution. In order to calculate the concentration of the silver solution needed, the following calculation is used: the concentration of silver solution is equal to the desired amount of silver per lens, multiplied by the dry weight of the lens divided by the total volume of treating solution.

$$\text{silver solution concentration } (\mu g/mL) = [\text{desired silver in lens } (\mu g/g) \times \text{average dry lens weight } (g)] / \text{total volume of treating solution } (mL)$$

For example, if one requires a lens containing 40 µg/g of silver, the dry weight of the lens is 0.02 g, and the vessel used to treat said lens has a volume of 3mL, the required silver concentration would be 0.27 µg/mL

It has been found that the ratio of the weight % silver to the weight % ligand in the lens should be greater than 0.6 and preferably greater than about 0.8. When ratios of the present invention are used, log reductions in microbial adhesion of at least about 0.4 logs (cfu/lens) and preferably greater than about 1 log (cfu/lens) may be achieved.

Silver solutions containing anywhere from about 0.10 µg/mL to 0.3 grams/mL may be used depending upon the concentration of the ligand monomer used to prepare the lenses of the invention. Aside from deionized water, other liquid media can be used such as water, aqueous buffered solutions and organic solutions such as polyethers or alcohols. Typically, the lens is washed in the silver solution for about 60 minutes, though the time may vary from about 1 minute to about 2 hours and at temperatures ranging from about 5°C to about 130°C. After the silver treatment the lenses are washed with several portions of water to obtain a lens where silver ions are releasably bound to the polymer via the ligand. The amount of silver that is incorporated into the lenses ranges from about 0.006 weight % (60 ppm) to 0.2. weight% (2,000 ppm), where any lens containing at least 60 ppm has the desired antimicrobial properties. The preferred amount of silver that is incorporated into the lens is 60 ppm to 1,000 ppm.

The term "antimicrobial" refers to a lens that exhibit one or more of the following properties - the inhibition of the adhesion of bacteria or other microbes to the lenses, the inhibition of the growth of bacteria or other microbes on the lenses, and the killing of bacteria or other microbes on the surface of the lenses or in a radius extending from the lenses (hereinafter adhesion of bacteria or other microbes to the lenses, the growth of bacteria or other microbes to the lenses and the presence of bacterial or other microbes on the surface of lenses is collectively referred to as "microbial production"). The lenses of the invention inhibit the microbial production by at least 0.4 log reduction (≥60% inhibition). Preferably, the lenses of the invention exhibit at least a 1-log reduction (≥ 90% inhibition) of viable bacteria or other microbes, bacteria or other microbes. Such bacteria or other microbes include but are not limited to those organisms found in the eye, particularly *Pseudomonas aeruginosa, Acanthamoeba species, Staphyloccus. aureus, E. coli, Staphyloccus epidermidis,* and *Serratia marcesens.* Preferably, said antimicrobial lens is a clear lens, that has clarity comparable to currently available commercial lenses such as but not limited to, etafilcon A, genfilcon A, lenefilcon A, polymacon, acquafilcon A, balafilcon A, galyfilcon, senofilcon and lotrafilcon A.

The advantages of the antimicrobial lenses of the invention are many. For example, other antimicrobial lenses that incorporate silver usually contain silver coordinated to some inorganic particulate matter. Often that particulate matter is visible to the naked or magnified eye, and it can affect the visual acuity of the user. However, the lenses of the invention do not have this problem. The ligand monomers are generally soluble with all of the other components of the antimicrobial lenses. Therefore when the lenses are produced they do not have substantial particulate matter due to their antimicrobial components. The antimicrobial lenses of the invention have comparable clarity to commercial lenses such as etafilicon A, genfilcon A, lenefilcon A, polymacon, acquafilcon A, balafilcon A, galyfilcon, senofilcon and lotrafilcon A.

Further, the invention includes a method of producing an antimicrobial lens comprising, silver and a polymer comprising at least one ligand monomer wherein the method comprises, consists essentially of, or consists of the steps of

(a) preparing a lens comprising at least one ligand monomer, and

(b) treating said lens with a silver solution in an amount sufficient to provide a silver to ligand monomer ratio of at least 0.6. The method is defined in claim 14.

The terms lens, antimicrobial, ligand monomer and silver all have their aforementioned meanings and preferred ranges. The term, "silver solution" refers to any liquid medium containing silver. The liquid medium includes but is not limited to water, deionized water, aqueous buffered solutions, alcohols, polyols, and glycols, where the preferred medium is deionized water. The silver of the solution is typically a silver salt such as silver nitrate, silver acetate, silver citrate, silver iodide, silver lactate, silver picrate, and silver sulfate. The concentration of silver in these solutions can vary from the concentration required to add a known quantity of silver to a lens to a saturated silver solution. The concentration of the silver solution needed, may be calculated as described above.

Silver solutions containing anywhere from about 0.10 μg/mL to 0.3 grams/mL have been used to prepare the lenses of the invention. Typically, the lens is washed in the silver solution for about 60 minutes, though the time may vary from about 1 minute to about 2 hours and at temperatures ranging from about 5°C to about 130°C. After the silver treatment the lenses are washed with several portions of water to obtain a lens where silver is incorporated into the polymer.

The lenses of the invention may be used in a method of reducing the adverse effects associated with microbial production in the eye of a mammal.

The phrase "adverse effects associated with microbial production" includes but is not limited to, ocular inflammation, contact lens related peripheral ulcers, contact lens associated red eye, infiltrative keratitis, and microbial keratitis.

In order to illustrate the invention the following examples are included. These examples do not limit the invention. They are meant only to suggest a method of practicing the invention. Those knowledgeable in contact lenses as well as other specialties may find other methods of practicing the invention. However, those methods are deemed to be within the scope of this invention.

## EXAMPLES

The following abbreviations were used in the examples

PVP= polyvinylpyrrolidinone;
MAA = methacrylic acid;
PAA = poly(acrylic acid)
ATU = allylthiourea;
Cell/prot = (Acrylamidomethyl)cellulose acetate propionate
3M3P = 3-methyl-3-propanol
D3O = 3,7-dimethyl-3-octanol
TAA = t-amyl alcohol
BAGE = glycerin esterified with boric acid
DI= deionized water;
PBS = phosphate-buffered saline, pH 7.4 ± 0.2;
TPBS = Phosphate-buffered saline with 0.05% Tween™ 80, pH 7.4 ± 0.2;
TSA = sterile tryptic soy agar;
TSB = sterile tryptic soy broth;
60% IPA = isopropyl alcohol, 60% v/v DI;
70% IPA = isopropyl alcohol, 70% v/v DI;
10% IPA = isopropyl alcohol, 10% v/v DI;
MVD = modified vortex device;
TBACB = tetrabutyl ammonium-m-chlorobenzoate
TMI = dimethyl meta-isopropenyl benzyl isocyanate
MMA = methyl methacrylate
HEMA = hydroxyethyl methacrylate
mPDMS = mono-methacryloxypropyl terminated polydimethylsiloxane MW = 800-1000
DMA = N,N-dimethylacrylamide
Blue HEMA = the reaction product of reactive blue number 4 and HEMA as described in Example 4 of U.S. Patent 5,944,853
DAROCUR 1173 = 2-hydroxy-2-methyl-1-phenyl-propan-1-one
EGDMA = ethyleneglycol dimethacrylate
TMPTMA = trimethyloyl propane trimethacrylate
TEGDMA = tetraethyleneglycol dimethacrylate

Norbloc = 2-(2'-hydroxy-5-methacrylyloxyethylphenyl)-2H-benzotriazole
CGI 1850 = 1:1 (w/w) blend of 1-hydroxycyclohexyl phenyl ketone and bis (2,6-dimethyoxybenzoyl)-2,4-4-trimethylpentyl phosphine oxide
w/w = weight/total weight
w/v = weight/total volume
v/v =volume/total volume
pHEMA = poly(hydroxyethyl) methacrylate coating as described in Example 14 of U.S. Serial No. 09/921,192, "Methods for Coating Articles by Mold Transfer"

The contact lenses of the invention were evaluated for antibacterial efficacy using the following biological assay: A culture of *Pseudomonas aeruginosa,* ATCC# 15442 (American Type Culture Collection, Rockville, MD), was grown overnight in a tryptic soy medium. The culture was washed three times in phosphate buffered saline ( PBS, pH = 7.4 +/- 0.2) and the bacterial pellet was resuspended in 10 ml of PBS. The bacterial inoculum was prepared to result in a final concentration of approximately 1 x $10^6$ colony forming units/mL (cfu/mL). Three contact lenses were rinsed in three changes of 30 milliliters of phosphate buffered saline (PBS, pH = 7.4 +/- 0.2) to remove residual solutions. Each rinsed lens was placed with 2 mL of the bacterial inoculum into a sterile glass vial, which was then rotated in a shaker-incubator (100 rpm) for two hours at 37 +/- 2°C. Each lens was removed from the glass vial, rinsed five times in three changes of PBS to remove loosely bound cells, placed into individual wells of a 24-well microtiter plate containing 1 mL PBS, and rotated in a shaker-incubator for an additional 22 hours at 37 +/- 2°C. Each lens was again rinsed five times in three changes of PBS to remove loosely bound cells, placed into 10 mL of PBS containing 0.05% (w/v) Tween™ 80, and vortexed at 2000 rpm for 3 minutes, employing centrifugal force to disrupt adhesion of the remaining bacteria to the lens. The resulting supernatant was enumerated for viable bacteria and the results of detectable viable bacteria attached to 3 lenses were averaged and this data is presented as the log reduction of the innoculum, as compared to control (lenses made from the Table 1 formulation without added silver).

Silver content was determined by Instrumental Neutron Activation Analysis "INAA". INAA is a qualitative and quantitative elemental analysis method based on the artificial induction of specific radionuclides by irradiation with neutrons in a nuclear reactor. Five lenses are placed individually in 20 ml polypropylene scintillation vials and dried in a vacuum oven at approximately 60°C for a minimum of 4 hours. The lenses were individually weighed and placed in irradiation vials and analyzed. Irradiation of the sample is followed by the quantitative measurement of the characteristic gamma rays emitted by the decaying radionuclides. The gamma rays detected at a particular energy are indicative of a particular radionuclide's presence, allowing for a high degree of specificity. Becker, D. A.; Greenberg, R.R.; Stone, S. F. J. Radioanal. Nucl. Chem. 1992, 160(1), 41-53; Becker, D. A.; Anderson, D. L.; Lindstrom, R. M.; Greenberg, R. R.; Garrity, K. M.; Mackey, E. A. J. Radioanal. Nucl. Chem. 1994, 179(1), 149-54. The INAA procedure used to quantify silver content in contact lens material uses the following two nuclear reactions:

1. In the activation reaction, $^{110}Ag$ is produced from stable $^{109}Ag$ (isotopic abundance = 48.16 %) after capture of a radioactive neutron produced in a nuclear reactor.
2. In the decay reaction, $^{110}Ag$ ($\tau^{1/2}$ = 24.6 seconds) decays primarily by negatron emission proportional to initial concentration with an energy characteristic to this radio- nuclide (657.8 keV).

The gamma-ray emission specific to the decay of $^{110}Ag$ from irradiated. standards and samples are measured by gamma-ray spectroscopy, a well-established pulse-height analysis technique, yielding a measure of the concentration of the analyte.

Th weight % ATU in the lenses is measured using HPLC. Three lenses weighed into a 20 ml glass scintillation vial and extracted with methanol. The extract is analyzed by HPLC using the following conditions:

Column: Prodigy ODS3 150 + 4.6 mm, 5 um particle diameter
mobile phase: 5% methanol 95% water
detector wavelength: 210 nm
injection volume: 10ul
flow rate: 1 ml/min

The amount of ATU in the extract is quantified by comparison of ATU peak area against external standards. The amount of ATU incorporated (i.e. co-polymerized) into the polymer is calculated by subtracting this value from the nominal concentration.

Example 1

To a dry container housed in a dry box under nitrogen at ambient temperature was added 30.0 g (0.277 mol) of bis(dimethylamino)methylsilane, a solution of 13.75 mL of a 1 M solution of TBACB (386.0 g TBACB in 1000 mL dry THF), 61.39 g (0.578 mol) of p-xylene, 154.28 g (1.541 mol) methyl methacrylate (1.4 equivalents relative to initiator), 1892.13 (9.352 mol) 2-(trimethylsiloxy)ethyl methacrylate (8.5 equivalents relative to initiator) and 4399.78 g (61.01 mol) of THF. To a dry, three-necked, round-bottomed flask equipped with a thermocouple and condenser, all connected to a nitrogen source, was charged the above mixture prepared in the dry box.

The reaction mixture was cooled to 15 °C while stirring and purging with nitrogen. After the solution reached 15 °C, 191.75 g (1.100 mol) of 1-trimethylsiloxy-1-methoxy-2-methylpropene (1 equivalent) was injected into the reaction vessel. The reaction was allowed to exotherm to approximately 62 °C and then 30 mL of a 0.40 M solution of 154.4 g TBACB in 11 mL of dry THF was metered in throughout the remainder of the reaction. After the temperature of reaction reached 30 °C and the metering began, a solution of 467.56 g (2.311 mol) 2-(trimethylsiloxy)ethyl methacrylate (2.1 equivalents relative to the initiator), 3636.6. g (3.463 mol) n-butyl monomethacryloxypropyl-polydimethylsiloxane (3.2 equivalents relative to the initiator), 3673.84 g (8.689 mol), TRIS (7.9 equivalents relative to the initiator) and 20.0 g bis (dimethylamino)methytsilane was added.

The mixture was allowed to exotherm to approximately 38-42 °C and then allowed to cool to 30 °C. At that time, a solution of 10.0 g (0.076 mol) bis(dimethylamino)methylsilane, 154.26 g (1.541 mol) methyl methacrylate (1.4 equivalents relative to the initiator) and 1892.13 g (9.352 mol) 2-trimethylsiloxy)ethyl methacrylate (8.5 equivalents relative to the initiator) was added and the mixture again allowed to exotherm to approximately 40 °C. The reaction temperature dropped to approximately 30 °C and 2 gallons of THF were added to decrease the viscosity. A solution of 439.69 g water, 740.6 g methanol and 8.8 g (0.068 mol) dichloroacetic acid was added and the mixture refluxed for 4.5 hours to de-block the protecting groups on the HEMA. Volatiles were then removed and toluene added to aid in removal of the water until a vapor temperature of 110 °C was reached.

The reaction flask was maintained at approximately 110 °C and a solution of 443 g (2.201 mol) TMI and 5.7 g (0.010 mol) dibutyltin dilaurate were added. The mixture was reacted until the isocyanate peak was gone by IR. The toluene was evaporated under reduced pressure to yield an off-white, anhydrous, waxy reactive monomer. The macromer was placed into acetone at a weight basis of approximately 2:1 acetone to macromer. After 24 hrs, water was added to precipitate out the macromer and the macromer was filtered and dried using a vacuum oven between 45 and 60 °C for 20-30 hrs.

Examples 2-4

Reactive monomer mixes were formed by dissolving the components, in the percentages listed in Table 1 and ATU in the amounts listed in Table 2, with D3O in an 80:20 weight % mixture as follows: the components listed in Table 1 and ATU were mixed with D3O in an Erlenmeyer flask, sonicated at approximately 45°C until all components are dissolved and were subsequently loaded into an eight cavity lens mold of the type described in U.S. Pat. No. 4,640,489 and cured for 30 minutes at 55°C. Polymerization occurred under a nitrogen purge and was photoinitiated with 5 mW $cm^{-2}$ visible light generated with a Philips TL 20W/03T fluorescent bulb. After curing, the molds were opened, and the lenses were released in a 60% IPA/water, then leached in IPA/DI to remove any residual monomers and diluent. Finally the lenses were equilibrated in either physiological borate-buffered saline or de-ionized water.

Table 1

| Component | Weight % |
|---|---|
| Macromer | 17.98 |
| TRIS | 14 |
| DMA | 26 |
| MPDMS | 28 |
| Norbloc | 2 |
| CGI 1850 | 1 |
| TEGDMA | 1 |
| HEMA | 5 |
| Blue HEMA | 0.02 |

(continued)

| Component | Weight % |
|---|---|
| PVP | 5 |

Examples 5-7

A stock solution of silver nitrate in DI water was prepared (1.0157g $AgNO_3$/100ml water). The $AgNO_3$ solution was diluted 1:100 in DI water. The lenses prepared in Examples 2-4 above were placed in glass vials with 3 ml special packing solution ("SPS" which contains the following in deionized $H_2O$: 0.18 weight % sodium borate [*1330-43-4*], Mallinckrodt and 0.91 weight % boric acid [*10043-35-3*], Mallinckrodt) per lens. Silver nitrate was added to each vial in a volume calculated to provide the desired silver to ATU ratio. The vials containing the lenses were autoclaved for 2 hours at 121°C. The treated lenses were removed from the silver solution and placed into distilled water (300 mL). The lenses were either rolled or stirred in distilled water for about 30 minutes. This water washing procedure was repeated three (3) more times. The resulting lenses were stored in saline solution and tested to determine their antimicrobial potential. The results of the bacterial adhesion assay are presented in Table 2, below. In addition, the lenses were analyzed by instrumental neutron activation analysis, to determine the amount of silver that was incorporated in the lenses. This data is presented in Table 2.

| Ex # | Ag (ppm) | ATU target (wt%) | ATU (wt%) | Log Redxn adhesion (cfu/lens) | [Ag]/[ATU] |
|---|---|---|---|---|---|
| 5 | 967±28 | 0.1 | 0.124 | 1.04±0.07 | 0.84 |
| 6 | 974±53 | 0.2 | 0.179 | 0.41±0.35 | 0.58 |
| 7 | 953+22 | 0.5 | 0.292 | 0.16±0.36 | 0.35 |

**Claims**

**1.** An antimicrobial lens comprising silver and a silicone elastomer or silicone hydrogel formed from a reaction mixture comprising at least one ligand monomer of Formula I

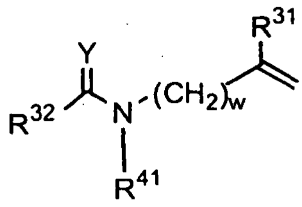

I

wherein

wis 0-1;
Y is oxygen or sulfur;
$R^{31}$ and $R^{41}$ are hydrogen; and

(i) $R^{32}$ is selected from the group consisting of amine, $C_{1-3}$alkylamine, phenylamine, substituted phenylamine, and thio$C_{1-3}$alkylcarbonyl; or
(ii) said monomer is selected from the following monomers:

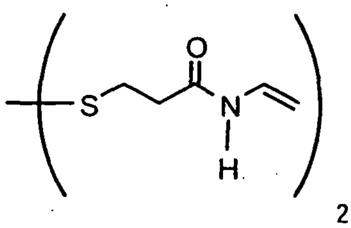, 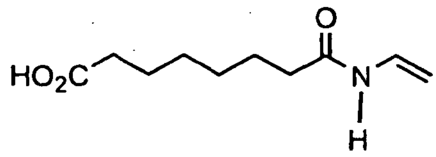,

and

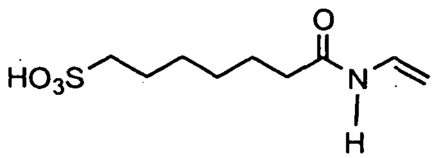

and wherein the silver is releasably bound to the ligand, and the silver is present in the lens in an amount, expressed as a weight ratio of silver to ligand monomer of at least 0.6, and at 60ppm by weight to 2000ppm by weight.

**2.** The antimicrobial lens of claim 1 wherein the lens is a soft contact lens.

**3.** The antimicrobial lens of claim 1 wherein the ligand monomer is present at 0.01 to 0.3 weight percent.

**4.** The antimicrobial lens of claim 1 wherein the ligand monomer is present at 0.01 to 0.2 weight percent.

**5.** The antimicrobial lens of claim 1 wherein the weight ratio of silver to ligand monomer is at least 0.8.

**6.** The antimicrobial lens of claim 1 wherein the lens is a silicone hydrogel.

**7.** The antimicrobial lens of claim 1 wherein, the lens is etafilcon A, balafilcon, A, acquafilcon A, lenefilcon A, galyfilcon, senofilcon or lotrafilcon A.

**8.** The antimicrobial lens of claim 1 wherein the monomer of Formula I is selected from the group consisting of 1-allyl-2 thiourea and the following monomers

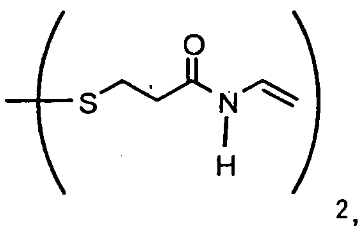, 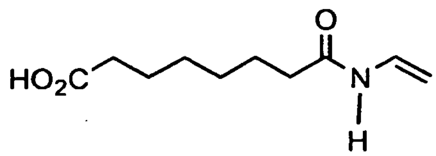,

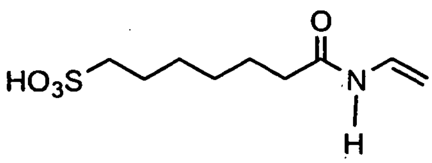, and 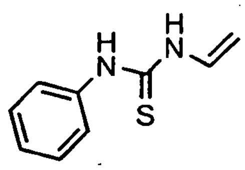.

**9.** The antimicrobial lens of claim 1 wherein silver is present at 60 ppm to 1000 ppm by weight.

**10.** The antimicrobial lens of claim 1 wherein the lens is a silicone hydrogel and the ligand monomer is 1-allyl-2-thiourea.

**11.** The antimicrobial lens of claim 1 wherein the lens is etafilcon A, balafilcon, A, acquafilcon A, lenefilcon, galyfilcon, senofilcon or lotrafilcon A and the ligand monomer is 1-allyl-2-thiourea.

**12.** The antimicrobial lens of claim 1 wherein the lens is etafilcon A or acquafilcon A.

**13.** The lens of claim 12 wherein silver is present at 60 ppm to 1000 ppm by weight.

**14.** A method of producing an antimicrobial lens comprising, silver and a silicone elastomer or silicone hydrogel comprising at least one ligand monomer of Formula I

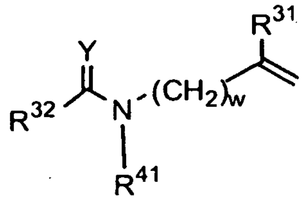

I .

wherein

w is 0-1;
Y is oxygen or sulfur;
$R^{31}$ and $R^{41}$ are hydrogen; and

(i) $R^{32}$ is selected from the group consisting of amine, $C_{1-3}$alkylamine, phenylamine, substituted phenylamine, and thio$C_{1-3}$alkylcarbonyl; or
(ii) said monomer is selected from the following monomers:

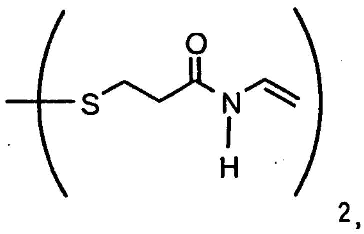

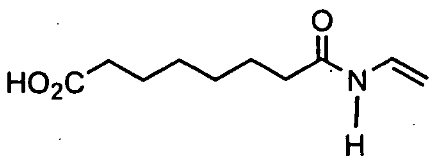

,

and

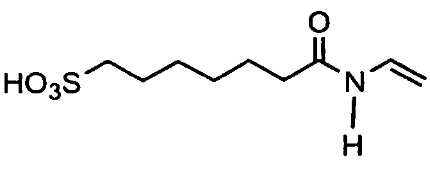

where the method comprises the steps of

(a) preparing a lens comprising at least one ligand monomer and
(b) treating the lens with a silver solution of a concentration to provide the lens with a silver to polymerized ligand monomer weight ratio of at least 0.6, and a silver content of 60 ppm by weight to 2000 ppm by weight.

**15.** The method of claim 14 wherein the silver solution is aqueous silver nitrate having a concentration of 0.1 μg/mL to 0.3 g/mL.

**16.** The method of claim 14 wherein the treating step comprises soaking the lens in the silver solution.

**17.** The method of claim 16 wherein the lens is soaked in the silver solution for 2 minutes to 2 hours.

**18.** The method of claim 15 wherein the treating step comprises storing the lens in a silver solution for 20 minutes to 5 years.

**19.** The method of claim 15 wherein the weight ratio of silver to ligand monomer is at least 0.8.

## Patentansprüche

**1.** Antimikrobielle Linse, die Silber und ein Silikon-Elastomer oder Silikon-Hydrogel umfasst, gebildet aus einer Reaktionsmischung, die wenigstens ein Ligandenmonomer von Formel I umfasst

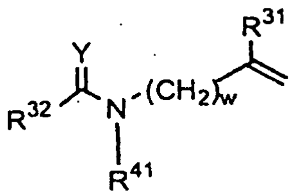

I

worin

w 0-1 ist;
Y Sauerstoff oder Schwefel ist;
$R^{31}$ und $R^{41}$ Wasserstoff sind; und

(i) $R^{32}$ ausgewählt ist aus der Gruppe, bestehend aus Amin, $C_{1-3}$-Alkylamin, Phenylamin, substituiertem Phenylamin und Thio-$C_{1-3}$-alkylcarbonyl; oder
(ii) das Monomer ausgewählt ist aus den folgenden Monomeren:

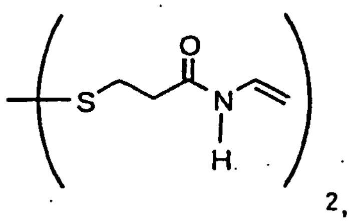

,

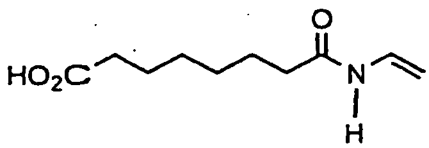

und

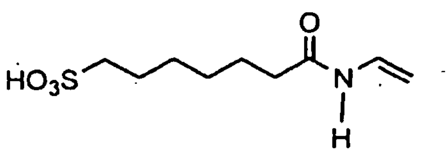

und wobei das Silber lösbar an den Liganden gebunden ist und das Silber in der Linse in einer Menge, ausgedrückt als ein Gewichtsverhältnis von Silber zu Ligandenmonomer, von wenigstens 0,6 und mit 60 ppm (gewichtsbezogen)

bis 2000 ppm (gewichtsbezogen) vorliegt.

**2.** Antimikrobielle Linse nach Anspruch 1, wobei die Linse eine weiche Kontaktlinse ist.

**3.** Antimikrobielle Linse nach Anspruch 1, wobei das Ligandenmonomer mit 0,01 bis 0,3 Gewichtsprozent vorliegt.

**4.** Antimikrobielle Linse nach Anspruch 1, wobei das Ligandenmonomer mit 0,01 bis 0,2 Gewichtsprozent vorliegt.

**5.** Antimikrobielle Linse nach Anspruch 1, wobei das Gewichtsverhältnis von Silber zu Ligandenmonomer wenigstens 0,8 beträgt.

**6.** Antimikrobielle Linse nach Anspruch 1, wobei die Linse ein Silikon-Hydrogel ist.

**7.** Antimikrobielle Linse nach Anspruch 1, wobei die Linse Etafilcon A, Balafilcon A, Acquafilcon A, Lenefilcon A, Galyfilcon, Senofilcon oder Lotrafilcon A ist.

**8.** Antimikrobielle Linse nach Anspruch 1, wobei das Monomer von Formel I ausgewählt ist aus der Gruppe, bestehend aus 1-Allyl-2-thioharnstoff und den folgenden Monomeren

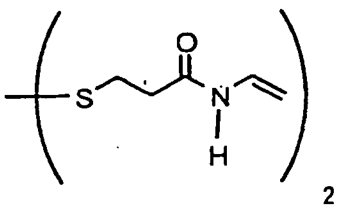,
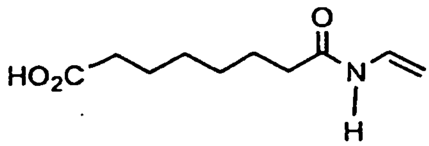,

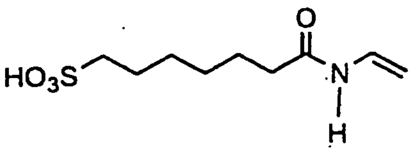
und
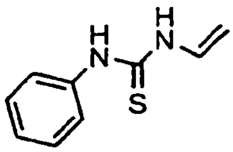.

**9.** Antimikrobielle Linse nach Anspruch 1, wobei Silber mit 60 ppm bis 1000 ppm (gewichtsbezogen) vorliegt.

**10.** Antimikrobielle Linse nach Anspruch 1, wobei die Linse ein Silikon-Hydrogel ist und das Ligandenmonomer 1-Ally-2-thioharnstoff ist.

**11.** Antimikrobielle Linse nach Anspruch 1, wobei die Linse Etafilcon A, Balafilcon A, Acquafilcon A, Lenefilcon, Galyfilcon, Senofilcon oder Lotrafilcon A ist und das Ligandenmonomer 1-Allyl-2-thioharnstoff ist.

**12.** Antimikrobielle Linse nach Anspruch 1, wobei die Linse Etafilcon A oder Acquafilcon A ist.

**13.** Linse nach Anspruch 12, wobei Silber mit 60 ppm bis 1000 ppm (gewichtsbezogen) vorliegt.

**14.** Verfahren zur Herstellung einer antimikrobiellen Linse, die Silber und ein Silikon-Elastomer oder Silikon-Hydrogel umfasst, das wenigstens ein Ligandenmonomer von Formel I umfasst

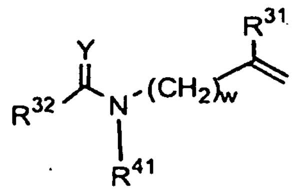

I

worin

w 0-1 ist;
Y Sauerstoff oder Schwefel ist;
$R^{31}$ und $R^{41}$ Wasserstoff sind; und

(iii) $R^{32}$ ausgewählt ist aus der Gruppe, bestehend aus Amin, $C_{1-3}$-Alkylamin, Phenylamin, substituiertem Phenylamin und Thio-$C_{1-3}$-alkylcarbonyl; oder
(iv) das Monomer ausgewählt ist aus den folgenden Monomeren:

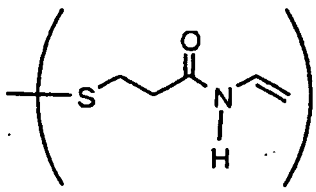

2,

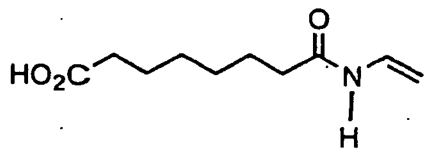

und

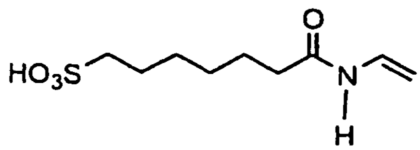

wobei das Verfahren die Schritte umfasst

(a) Herstellung einer Linse, die wenigstens ein Ligandenmonomer umfasst, und
(b) Behandeln der Linse mit einer Silberlösung mit einer Konzentration, um die Linse mit einem Gewichtsverhältnis von Silber zu polymerisiertem Ligandenmonomer von wenigstens 0,6 und einem Silbergehalt von 60 ppm (gewichtsbezogen) bis 2000 ppm (gewichtsbezogen) zu versehen.

**15.** Verfahren nach Anspruch 14, wobei die Silberlösung wässriges Silbernitrat mit einer Konzentration von 0,1 μg/ml bis 0,3 μg/ml ist.

**16.** Verfahren nach Anspruch 14, wobei der Behandlungsschritt das Einweichen der Linse in der Silberlösung umfasst.

**17.** Verfahren nach Anspruch 16, wobei die Linse in der Silberlösung für 2 Minuten bis 2 Stunden eingeweicht wird.

**18.** Verfahren nach Anspruch 15, wobei der Behandlungsschritt die Lagerung der Linse in einer Silberlösung für 20 Minuten bis 5 Jahre umfasst.

**19.** Verfahren nach Anspruch 15, wobei das Gewichtsverhältnis von Silber zu Ligandenmonomer wenigstens 0,8 beträgt.

## Revendications

1. Lentille antimicrobienne comprenant de l'argent et un élastomère de silicone ou un hydrogel de silicone formé à partir d'un mélange réactionnelle comprenant au moins un ligand monomère de formule I

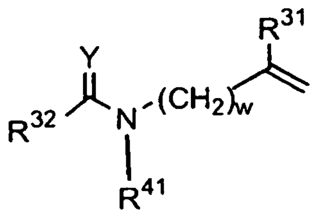

I

dans laquelle

w est égal à 0-1 ;
Y est un atome d'oxygène ou de soufre ;
$R^{31}$ et $R^{41}$ sont de l'hydrogène ; et

(i) $R^{32}$ est choisi dans le groupe constitué d'amine, d'alkylamine en $C_{1-3}$, de phénylamine, de phénylamine substituée, et de thio-alkylcarbonyle en $C_{1-3}$ ; ou
(ii) ledit monomère est choisi parmi les monomères suivants :

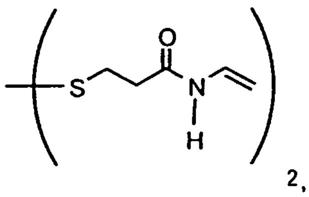
,
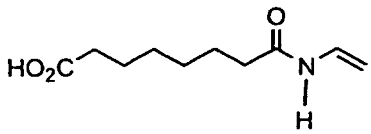
,

et

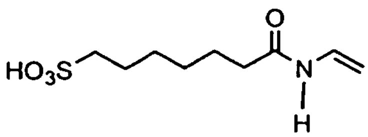

et dans laquelle l'argent est lié de manière amovible au ligand, et l'argent est présent dans la lentille dans une quantité, exprimée comme le rapport en poids de l'argent sur ligand monomère, d'au moins 0,6 et de 60 ppm en poids à 2 000 ppm en poids.

2. Lentille antimicrobienne selon la revendication 1, dans laquelle la lentille est une lentille de contact souple.

3. Lentille antimicrobienne selon la revendication 1, dans laquelle le ligand monomère est présent de 0,01 à 0,3 pour cent en poids.

4. Lentille antimicrobienne selon la revendication 1, dans laquelle le ligand monomère est présent de 0,01 à 0,2 pour cent en poids.

5. Lentille antimicrobienne selon la revendication 1, dans laquelle le rapport en poids de l'argent sur le ligand monomère est d'au moins 0,8.

6. Lentille antimicrobienne selon la revendication 1, dans laquelle la lentille est un hydrogel de silicone.

7. Lentille antimicrobienne selon la revendication 1, dans laquelle la lentille est en étafilcon A, balafilcon A, acquafilcon A, lènefilcon A, galyfilcon, sénofilcon ou lotrafilcon A.

8. Lentille antimicrobienne selon la revendication 1, dans laquelle le monomère de Formule I est choisi dans le groupe constitué du 1-allyl-2-thiourée et des monomères suivants

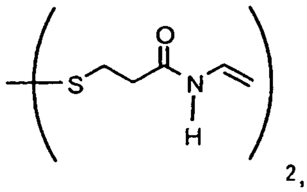
,
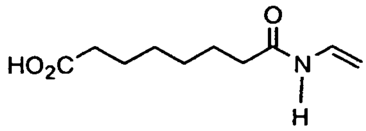
,

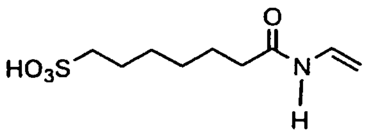
, et
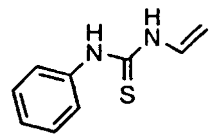
.

9. Lentille antimicrobienne selon la revendication 1, dans laquelle l'argent est présent de 60 ppm à 1 000 ppm en poids.

10. Lentille antimicrobienne selon la revendication 1, dans laquelle la lentille est un hydrogel de silicone et le ligand monomère est une 1-allyl-2-thiourée.

11. Lentille antimicrobienne selon la revendication 1, dans laquelle la lentille est en étafilcon A, en balafilcon A, en acquafilcon A, en lènefilcon, en galyfilcon, en sénofilcon, ou en lotrafilcon A et le ligand monomère est de la 1-allyl-2-thiourée.

12. Lentille antimicrobienne selon la revendication 1, dans laquelle la lentille est l'étafilcon A ou l'acquafilcon A.

13. Lentille selon la revendication 12, dans laquelle de l'argent est présent de 60 ppm à 1 000 ppm en poids.

14. Méthode de production d'une lentille antimicrobienne comprenant de l'argent et un élastomère de silicone ou un hydrogel de silicone comprenant au moins un ligand monomère de formule I

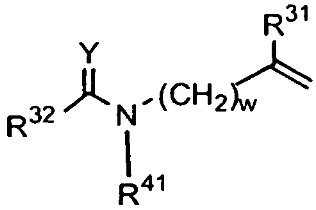

I

dans laquelle

w est égal à 0-1 ;
Y est un atome d'oxygène ou de soufre ;
$R^{31}$ et $R^{41}$ sont de l'hydrogène ; et

(i) $R^{32}$ est choisi dans le groupe constitué d'amine, d'alkylamine en $C_{1-3}$, de phénylamine, de phénylamine substituée, et de thio-alkylcarbonyle en $C_{1-3}$ ; ou

(ii) ledit monomère est choisi parmi les monomères suivants :

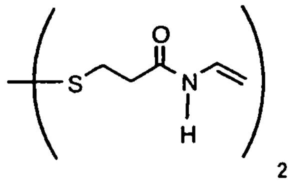

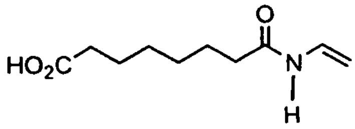,

et

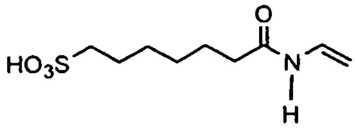

où la méthode comprend les étapes consistant à :

(a) préparer une lentille comprenant au moins un ligand monomère et
(b) traiter la lentille avec une solution d'argent ayant une concentration permettant de doter la lentille d'un rapport en poids d'argent sur ligand monomère polymérisé d'au moins 0,6, et une teneur en argent de 60 ppm en poids à 2 000 ppm en poids.

**15.** Méthode selon la revendication 14, dans laquelle la solution d'argent est un nitrate d'argent aqueux ayant une concentration de 0,1 μg/ml à 0,3 g/ml.

**16.** Méthode selon la revendication 14, dans laquelle la phase de traitement comprend l'immersion de la lentille dans la solution d'argent.

**17.** Méthode selon la revendication 16, dans laquelle la lentille est immergée dans la solution d'argent pendant 2 minutes à 2 heures.

**18.** Méthode selon la revendication 15, dans laquelle la phase de traitement comprend le stockage de la lentille dans une solution d'argent pendant 20 minutes à 5 ans.

**19.** Méthode selon la revendication 15, dans laquelle le rapport en poids d'argent sur le ligand monomère est d'au moins 0,8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0249683 A
- US 6367929 B
- WO 03022321 A
- WO 03022322 A
- US 3808178 A
- US 4120570 A
- US 4136250 A
- US 4153641 A
- US 4740533 A
- US 5034461 A
- US 5070215 A
- US 5998498 A
- US 5760100 A
- US 5260000 A
- US 4719943 A
- US 4139593 A
- US 4139548 A
- US 4235985 A
- EP 080539 A
- US 5710302 A
- WO 9421698 A
- EP 406161 A
- JP 2000016905 B
- WO 5760100 A
- WO 5260000 A
- US 6087415 A
- US 5776999 A
- US 5849811 A
- US 5789461 A
- WO 0302232 A
- WO 10236762 A
- US 3854982 A
- US 3916033 A
- US 4920184 A
- US 5002794 A
- US 5712327 A
- WO 0127662 A
- WO 03011551 A
- US 5453467 A
- US 5422402 A
- WO 9300391 A
- US 4973493 A
- US 5350800 A
- US 5944853 A
- US 09921192 B
- US 4640489 A


### Non-patent literature cited in the description


- Photoinitiators for Free Radical Cationic & Anionic Photopolymerization. John Wiley and Sons, 1998, vol. III
- **BECKER, D. A. ; GREENBERG, R.R. ; STONE, S. F. J.** *Radioanal. Nucl. Chem.,* 1992, vol. 160 (1), 41-53
- **BECKER, D. A. ; ANDERSON, D. L. ; LINDSTROM, R. M. ; GREENBERG, R. R. ; GARRITY, K. M. ; MACKEY, E. A.** *J. Radioanal. Nucl. Chem.,* 1994, vol. 179 (1), 149-54